# EUROPÄISCHE PATENTSCHRIFT

(11) **EP 2 132 994 B1**
(45) Veröffentlichungstag und Bekanntmachung des Hinweises auf die Patenterteilung: **26.05.2010**
(21) Anmeldenummer: 08010645.3
(22) Anmeldetag: 11.06.2008
(51) Int. Cl.: A23L 1/30, A23L 2/02, A23L 2/52, A23L 2/38

(54) **Fermentation von Granatapfelsaft-haltigen Lösungen durch Saccharomyces boulardii und Lactobacillen, daraus erhältliche Fermentationsprodukte und deren Verwendung**
Fermentation of solutions containing pomegranate juice with saccharomyces boulardii and lactobacilli, resulting fermented products and application thereof
Fermentation de solutions contenant du jus de grenade à l'aide de saccharomyces boulardii et de lactobaciles, produits de fermentation ainsi obtenus et leur utilisation

(43) Veröffentlichungstag der Anmeldung: 16.12.2009
(73) Patentinhaber: Jacob, Ludwig Manfred, 55262 Heidesheim (DE)
(72) Erfinder: Jacob, Ludwig Manfred, 55262 Heidesheim (DE)
(74) Vertreter: Leifert & Steffan

(56) Entgegenhaltungen:
- WO-A-00/57726
- WO-A-01/37848
- WO-A-2007/004229
- CN-A- 101 120 787
- US-A1- 2003 235 559
- US-A1- 2008 044 504
- DATABASE WPI Week 200359 Thomson Scientific, London, GB; AN 2003-621352 XP002507767 & JP 2003 116497 A (HONGO F) 22. April 2003 (2003-04-22)

## Beschreibung

Die Erfindung betrifft polyphenolreiche Produkte aus Pflanzen sowie ein Verfahren zur Herstellung dieser Produkte. Des weiteren betrifft die Erfindung die Verwendung der polyphenolhaltigen Produkte zur Herstellung von Lebensmitteln, Nahrungsergänzungsmitteln, diätetischen Lebensmitteln und Kosmetika; sowie Lebensmittel, Nahrungsergänzungsmittel, diätetische Lebensmittel und Kosmetika, die ein solches Produkt enthalten.

Polyphenole bilden eine Gruppe der sekundären Pflanzeninhaltsstoffe. Sie kommen im Pflanzenreich ubiquitär mit über 8000 Verbindungen vor und lassen sich in ihrer Grundstruktur von Phenol (Hydroxybenzol) ableiten. Die Grundstruktur der Polyphenole besteht aus einem aromatischen Ring oder Ringsystem mit zwei oder mehr direkt daran gebundenen Hydroxylgruppen. Als Polyphenole werden somit aromatische Verbindungen verstanden, die mindestens zwei phenolische Hydroxygruppen im Molekül enthalten. Hierzu zählen insbesondere die drei Dihydroxybenzole (Brenzkatechin, Resorcin, Hydrochinon) und ihre Derivate. In der Natur treten beispielsweise freie und veretherte Polyphenole in Blütenfarbstoffen (z.B. Anthocyane, Anthocyanidine, Flavone, Flavonoide), in Gerbstoffen (z.B. Catechine, Tannine), als Flechten- oder Farn-Inhaltsstoffe (z.B. Usninsäure, Acylpolyphenole), in Ligninen, als Gallussäure-Derivate, Phloroglucin, Pyrogallol oder Hexahydroxybenzol auf. Im sauren Milieu sind die Polyphenole üblicherweise gut wasserlöslich. Sie sind sehr oxidationsanfällig und bilden im Zuge der Polymerisation und Oxidation braun gefärbte Niederschläge. Unter Berücksichtigung ihrer chemischen Struktur umfassen die Polyphenole die Gruppen der Phenolcarbonsäuren, der Flavonoide, der Anthocyane bzw. Anthocyanidine und der Stilbenderivate. Polyphenole können in Form von höhermolekularen Verbindungen, wie beispielsweise als Glykoside oder Ester, vorkommen. Sehr häufig liegen Polyphenole in der Pflanze als Glykoside (an einen oder mehrere, gleiche oder verschiedene Zuckermonomere gebunden) oder als Polymere vor.

Die Polyphenole werden den sogenannten "sekundären Pflanzeninhaltsstoffen" zugeordnet, da sie im Primärstoffwechsel der Pflanze nicht synthetisiert oder metabolisiert werden. Die genauen Biosynthese- sowie Metabolisierungswege der sekundären Pflanzeninhaltsstoffe sind noch nicht vollständig aufgeklärt. Bewiesenermaßen besitzen sie jedoch hormonellen (Wuchsstoffe, Steroide und Pheromone) und protektiven Charakter, wobei insbesondere der Schutz vor UV-Strahlung, Fraßfeinden und Pilz- oder Bakterienbefall zu nennen sind. Einige der sekundären Pflanzeninhaltsstoffe, wie Flavonoide und Anthocyane, tragen auch maßgeblich zur Farbe von Blüten und/oder Früchten bei.

Es sind zahlreiche Pflanzen mit einem hohen Gehalt an Polyphenolen bekannt. Dazu zählen unter anderem in Europa geläufige Pflanzen wie Äpfel, Tee, die Blätter und Trauben roter Weinreben, Himbeeren, Erdbeeren, Holunderbeeren, Schlehen, schwarze Johannisbeeren und schwarze Kirschen, als auch weniger geläufige Pflanzen und Pflanzenteile, wie die Schale und das Fruchtfleisch der Mangostanfrucht (Garcinia mangostana), Acai-Beeren, Amlabeeren, Aroniabeeren, Granatäpfel, Gingko, der Samen von Perilla (Perilla frutescens), Schwarznessel, chinesische Zitronenmelisse und wilder Sesam. Die in der Pflanze vorhandenen Polyphenole liegen dabei ganz überwiegend als sogenannte Glykoside an einen oder mehrere, gleiche oder verschiedene Zuckermoleküle gebunden und/oder als höhermolekulare Polymere vor.

Das zunehmende Interesse an Polyphenolen resultiert aus verschiedenen in vivo- und in vitro-Studien, in denen unter anderem antikanzerogene, antimutagene, antioxidative, antivirale, antiproliferative, antithrombotische und lipidsenkende Effekte nachgewiesen wurden.

Die Aufnahme von Polyphenolen in den Körper erfolgt ganz überwiegend durch Resorption über die Darmschleimhaut. Dabei können grundsätzlich niedermolekulare Verbindungen die Darmschleimhaut regelmäßig sehr viel besser passieren als höhermolekulare Verbindungen. Da die pflanzlichen Polyphenole überwiegend als Glykoside oder höhermolekulare Polymere vorliegen, müssen für eine Resorption der Polyphenole zunächst die Zuckerbindungen der Glykoside gespalten und die reinen, nicht an Zucker gebundenen Polyphenole (Aglyka) freigesetzt werden bzw. die höhermolekularen Polymere der Phenolsäuren hydrolysiert werden.

Dies leistet beim Menschen im wesentlichen die Dickdarmflora mit einer Vielzahl verschiedener Enzyme. Teilweise werden Glykoside auch bereits im Dünndarm durch das Enzym Laktasephlorizinhydrolase auf dem Bürstensaum der Dünndarmzellen hydrolysiert. Jedoch weist die Dünndarmflora von etwa 5% der Europäer und etwa 90% der Afrikaner und Asiaten einen Mangel an diesem Enzym auf. Eine der Laktasephlorizinhydrolase ähnliche Funktion besitzt die beta-Glukosidase der Dünndarmzellen, die allerdings intrazellulär (cytosolisch) vorliegt. Der Hauptanteil der Glykoside fällt daher ungespalten im Dickdarm an. Viele Glykoside lassen sich somit erst durch das Dickdarmmikrobiom, d.h. beispielsweise durch spezifische Glukosidasen im Colon, spalten (Scalbert und Williamson, Dietary intake and bioavailability of polyphenols, J. Nutr. 2000 Aug; 130(8S Suppl): 2073S-85S).

Polyphenole werden daher vorwiegend durch die Enzyme der Dickdarmflora aus ihrer glykosidischen bzw. polymeren Form freigesetzt. Zahlreiche Polyphenole unterliegen zudem einem enterohepatischen Kreislauf als Glucuronide, Methylate oder Sulfate, welche zum Teil ebenfalls durch das Dickdarmmikrobiom gespalten werden. Die durch die Metabolisierung im Dickdarm freiwerdenden Polyphenol-Aglyka weisen nicht nur ein geringeres Molekulargewicht auf, sondern sind auch lipophiler als die Glykoside. Aus diesen Gründen können die Aglyka die hydrophobe Zellmembran der Colozyten in signifikantem Ausmaß passieren und in den Blutkreislauf aufgenommen werden.

Die Bioverfügbarkeit der Polyphenol-Aglyka hängt also entscheidend von der Stoffwechselaktivität der Dickdarmflora ab. Eine ausreichende Bioverfügbarkeit der Polyphenole ist dann gegeben, wenn eine adäquate Dickdarmflora vorhanden ist, die zum einen die Zuckerbindung der Polyphenol-Glykoside spaltet und die Polyphenol-Aglyka freisetzt, und zum anderen die höhermolekularen Polyphenol-Polymere in niedermolekulare Bestandteile metabolisiert, sowie gegebenenfalls durch eine weitere Biotransformation zu noch lipophileren Molekülen die Bioverfügbarkeit der Polyphenole zusätzlich erhöht. Nur bei ausreichend hoher Bioverfügbarkeit werden Polyphenole in einer solchen Menge in den Blutkreislauf aufgenommen, dass eine ausreichend hohe Wirkkonzentration erreicht wird. Somit wird die biologische Wirkung der Polyphenole unmittelbar durch die Stoffwechselaktivität und Zusammensetzung der Dickdarmflora beeinflußt.

Die Zusammensetzung und metabolische Aktivität des Dickdarmmikrobioms weist jedoch starke interindividuelle und auch speziesspezifische Unterschiede auf. Beispielsweise kann das Dickdarmmikrobiom von Mäusen bestimmte GranatapfelPolyphenole nicht fermentieren. Auch beim Menschen ergeben sich erhebliche interindividuelle Unterschiede in der Zusammensetzung und metabolischen Aktivität der Darmflora. Die Bioverfügbarkeit und damit die biologische Wirkung von Pflanzeninhaltsstoffen, insbesondere auch von Polyphenolen, kann daher für verschiedene Personen sehr unterschiedlich ausfallen.

Es sind verschiedene Vorgänge bekannt, bei denen Pflanzeninhaltsstoffe auch außerhalb des menschlichen Körpers metabolisch umgesetzt werden. So stellt beispielsweise das Verfaulen von Fallobst eine durch Mikroorganismen verursachte Biotransformation von Inhaltsstoffen des Fallobstes dar. Auch die Herstellung von Wein oder Bier unter Einsatz von Wein- oder Bierhefe stellt einen solchen Prozeß dar.

Die Patentschrift US 5,639,496 A beschreibt Nahrungsmittel, die durch Fermentation mit Saccharomyces boulardii erhalten wurden, insbesondere aus Getreide wie Weizen und Mais, Soja und verschiedenen Gemüsen wie z.B. Erbsen.

Die Patentschrift US 5,891,440 beschreibt unter anderem die Herstellung einer Hautcreme aus verschiedenen pflanzlichen Bestandteilen, darunter Öl aus Granatapfelsamen, Kokosmilch, chinesischem Spargel und Schizandra-Beeren. Ein Teil des Granatapfelsamenöls kann alternativ durch eine Mischung aus trockenem Rotwein, vorzugsweise Carmel Hilonim 1995 der Carmel Winery, Israel, und mit Weinhefe fermentierten Granatapfelsamen und -saft ersetzt werden.

Die Patentschrift US 6,953,574 B2 beschreibt die Fermentation von unterschiedlichsten Bestandteilen verschiedenster Pflanzen, wie verschiedener Gemüse- oder Obstsorten, Beeren, Kräuter, Pilze, Nüsse, sowie tierischer Produkte, wie proteinreicher Organe oder Imkereiprodukte, durch verschiedenste Mikroorganismen wie Bifidobakterien, Essigsäure- oder Propionsäurebakterien, Hefen, Laktobazillen oder auch Pilze. Es werden unterschiedlichste hydrolysierte fermentierte Medien erhalten, die in unterschiedlichem Ausmaß Stoffwechselprodukte verschiedener Mikroorganismen enthalten. Das Einsatzgebiet der allgemein beschriebenen Medien wird sehr breit angegeben und umfaßt offenbar die Behandlung nahezu aller Erkrankungen.

Von besonderem Interesse für die adjuvante Ernährungstherapie, ernährungsphysiologische Prävention, Behandlung und/oder Prophylaxe von Krankheiten sind jedoch Produkte, die durch Herstellungsverfahren mit ganz konkret beschriebenen und gezielt ausgewählten Ausgangsstoffen erhalten werden können, ein geeignetes Spektrum von Inhaltsstoffen aufweisen und sich daher gezielt zum Einsatz zur allgemeinen Gesunderhaltung sowie bei bestimmten Erkrankungen zur adjuvanten Ernährungstherapie und ernährungsphysiologischen Prävention eignen.

Aufgabe der vorliegenden Erfindung war es daher, ein Produkt mit einem hohen Gehalt an gut bioverfügbaren Polyphenolen bereitzustellen, das in ernährungsphysiologischer Dosierung zur Herstellung von Lebensmitteln, Nahrungsergänzungsmitteln sowie diätetischen Lebensmitteln zur allgemeinen Gesunderhaltung, insbesondere von Herz, Blutgefäßen und Prostata, und in pharmakologischer Dosierung als Arzneimittel, insbesondere zur adjuvanten Therapie und Prävention von Krebserkrankungen (insbesondere Prostata-, Mamma-, Colon- und/oder Lungenkarzinom; insbesondere auch unterstützend und synergistisch bei einer Chemotherapie und/oder Strahlentherapie), benignen Prostatahyperplasie, arteriosklerotischen Gefäßveränderungen, koronarer Herzkrankheit, Zerebralsklerose, Hypercholesterinämie, chronischen entzündlichen Erkrankungen wie z.B. Rheumatoider Arthritis; Morbus Alzheimer, Morbus Parkinson, Multipler Sklerose und/oder Mikro- und Makroangiopathien bei Diabetes mellitus, geeignet ist.

Die Aufgabe wird überraschenderweise gelöst durch ein Fermentationsprodukt, erhältlich durch ein Verfahren umfassend die Fermentation eines Fermentationsansatzes enthaltend Granatapfelsaft, wobei zur Fermentation die Hefe Saccharomyces boulardii und mindestens eine Art von Lactobacillen, ausgewählt aus der Gruppe bestehend aus den Arten Lactobacillus plantarum, Lactobacillus paraplantarum, Lactobacillus pentosus und Lactobacillus acidophilus, eingesetzt wird.

Der Granatapfel (Frucht des Granatapfelbaums, *Punica granatum*) ist eine der ältesten Kultur- und Gesundheitsfrüchte der Menschheit. In den letzten Jahren ist der Granatapfel auch in den Blickpunkt der Forschung gerückt. Etwa 250 Veröffentlichungen in anerkannten wissenschaftlichen Fachjournalen sind zum Granatapfel bislang erschienen. In vitro, in vivo und in klinischen Studien werden dem Granatapfelsaft dabei überzeugende antioxidative, antiinflammatorische, antiarteriosklerotische und anticancerogene Wirkungen nachgewiesen. In einer Studie mit 48 Prostatakrebs-Patienten zeigte sich bei regelmäßiger Einnahme von Granatapfelsaft u.a. eine Vervierfachung der Verdoppelungszeit des Blutspiegels des Prostatakrebs-Markers PSA (prostataspezifisches Antigen), was für eine starke Verlangsamung des Fortschreitens der Prostatakrebserkrankung spricht (Pantuck et al., Phase II Study of Pomegranate Juice for Men with Rising Prostate-Specific Antigen following Surgery or Radiation for Prostate Cancer, Clin. Cancer Res. 2006; 12, 13: 4018-4026). Andere Studien wiesen Granatapfelsaft im Tiermodell und in vitro eine ausgeprägte wachstumshemmende Wirkung beispielsweise gegen Lungen-, Darm-, Haut- und Brustkrebs nach. Die Einnahme von Granatapfelsaft verbesserte in klinischen Studien deutlich die Durchblutung des Myokards (Herzmuskels) bei Patienten mit koronarer Herzkrankheit (KHK) und reduzierte arteriosklerotische Ablagerungen in der Halsschlagader (*Arteria carotis*)*.* NF-kappaB- und TNF-alpha-vermittelte Entzündungsprozesse und die Entwicklung der Alzheimer-Demenz wurden gehemmt. Hierfür verantwortlich scheinen nicht bestimmte isolierte Verbindungen zu sein, sondern ein synergistisches Zusammenwirken verschiedener natürlicher Inhaltsstoffe des Granatapfels. Diese modulieren intrazelluläre Signalwege sowie die Genexpression und Genregulation.

Der Granatapfel weist einen sehr hohen Gehalt an bestimmten sekundären Pflanzenstoffen, den Polyphenolen, auf. Dazu zählen insbesondere Phenolcarbonsäuren (Gallussäure, Ellagsäure), deren polymerisierte Derivate (vor allem Ellagitannine, wie z.B. Punicalagin), Flavonoide (wie beispielsweise Catechin, Quercetin, Rutin) sowie Anthocyane (wie Delphidin-3,5-diglukosid, Delphidin-3-diglukosid, Cyanidin-3,5-diglukosid, Cyanidin-3-glukosid, Pelargonidin-3-glukosid).

Es ist bekannt, dass bestimmte Granatapfelprodukte Polyphenole, beispielsweise in Form von Phenolcarbonsäuren und deren Polymeren sowie Flavonoiden, enthalten können. Die Polyphenole des Granatapfels liegen grundsätzlich überwiegend als Glykoside oder höhermolekulare Polymere vor. Granatapfelprodukte werden bislang entweder als Presssaft aus dem Granatapfelmark oder als getrocknete Extrakte aus den Schalen und Membranen des Granatapfels angeboten. Zur Herstellung werden dabei mechanische, wäßrige und/oder alkoholische Extraktionsweisen verwendet. Bei allen diesen Verfahren verbleiben jedoch die Polyphenole in ihrer Zuckerbindung, d.h. als Glykoside, bestehen bzw. liegen nach wie vor als höhermolekulare Polymere im Granatapfelprodukt vor. Ihre direkte Bioverfügbarkeit ist damit niedrig und erfordert erst eine Biotransformation durch das Darmmikrobiom. Gleichzeitig hängt die Bioverfügbarkeit der Aglyka bzw. niedermolekularen Spaltprodukte sehr stark von der individuellen Dickdarmflora des Patienten ab.

So hat sich in der bisher einzigen und aufsehenerregenden Studie hinsichtlich der oralen Anwendung von Granatapfelsaft bei Prostatakrebs-Patienten herausgestellt, dass zwar über 80% der Studienteilnehmer auf die Behandlung mit Granatapfelsaft ansprachen ("Responder"). Dies zeigte sich in einer Verlängerung der Verdoppelungszeit für den Blutspiegel des Prostatakrebs-Markers PSA von durchschnittlich 15 Monaten auf durchschnittlich 54 Monate, was für eine sehr deutliche Verzögerung des Fortschreitens der Erkrankung spricht. Jedoch gab es unter den Respondern sehr deutliche Unterschiede in der Ausprägung dieser Wirkung. Dies spiegelt sich in der großen Schwankungsbreite der erzielten PSA-Verdopplungszeit von 54 ± 102 Monaten wider. Bei einem Teil der Patienten wurde nur eine leichte Verzögerung des Anstiegs des PSA-Blutspiegels erreicht, während bei 35% der Patienten sogar zu einer absoluten Absenkung des PSA-Blutspiegels kam. Bei 9% der Patienten wurde ein Abfall des PSA-Spiegels um über 50% beobachtet (Pantuck et al., 2006). Diese hohe Schwankungsbreite in der Wirkung des Granatapfelsafts läßt sich nicht allein durch mögliche Unterschiede der Prostatakarzinome erklären, sondern ist auch auf interindividuelle Unterschiede in der Bioverfügbarkeit der Granatapfelpolyphenole zurückzuführen. Dies wird eindrucksvoll durch eine Bioverfügbarkeitsstudie mit Granatapfelpolyphenolen belegt. Nach oraler Einnahme zeigte es sich, dass die Serumkonzentrationen der Wirkstoffe stark zwischen verschiedenen Personen schwankten und die untersuchten Metabolite bei einem Drittel der Probanden überhaupt nicht im Blut auftauchten. Dieses Phänomen wurde in erster Linie auf die individuelle Zusammensetzung der Dickdarmflora zurückgeführt (Cerdä et al., J. Agric. Food Chem. 2005 Jul 13; 53(14): 5571-6).

Gesichert ist, dass die biologische Wirkung des Granatapfelsafts zum Großteil den aus den glykosidischen und polymeren Polyphenolen entstandenen aglykonen Metaboliten der Darmflora zuzuschreiben ist, nicht einer direkten Wirkung der im Saft enthaltenen glykosidischen und polymeren Polyphenole. Dies wird auch durch die Ergebnisse der Bioverfügbarkeitsstudien gestützt.

Des weiteren scheint es für die besondere Wirkung des Granatapfelsafts nicht auf einen oder wenige Einzelstoffe, sondern auf die synergistische Wirkung eines Spektrums von Inhaltsstoffen anzukommen (L.M. Jacob, Granatapfel: Prävention und adjuvante Ernährungstherapie bei Krebserkrankungen, EHK 2007 (56): 464-473). Insgesamt scheint kein einzelner, konkreter pharmakologischer Wirkmechanismus vorzuliegen, sondern eher eine positive ernährungsphysiologische Wirkung des Granatapfelsafts.

Insbesondere scheinen auch verschiedene aglykone Polyphenole an der Wirkung beteiligt zu sein, die erst durch Biotransformation bzw. Deglykosylierung von Granatapfelinhaltsstoffen entstehen bzw. freigesetzt und damit bioverfügbar werden.

Um ein Produkt mit einem hohen Gehalt an gut bioverfügbaren Polyphenolen bereitzustellen, wird erfindungsgemäß daher nicht nur zunächst der Granatapfelsaft als polyphenolreiches Ausgangsprodukt eingesetzt, sondern darin enthaltene Inhaltsstoffe werden zusätzlich bereits durch eine - außerhalb des menschlichen Körpers stattfindende - Fermentation mit geeigneten Mikroorganismen biotransformiert und bioverfügbar gemacht. Dies ermöglicht es auch Personen mit individuell hierzu weniger geeigneter Darmflora, von den wirksamen Polyphenolmetaboliten zu profitieren.

Erfindungsgemäß werden zur Fermentation die Hefe Saccharomyces boulardii und mindestens eine Art von Lactobacillen, ausgewählt aus der Gruppe bestehend aus den Arten Lactobacillus plantarum, Lactobacillus paraplantarum, Lactobacillus pentosus und Lactobacillus acidophilus, eingesetzt.

Die Hefe Saccharomyces boulardii zählt zur Gattung der Zuckerhefen (Saccharomyces) und wurde erstmals 1923 in Indochina von Henri Boulard auf Schalen von Lychee-Früchten nachgewiesen. Das Vorhandensein der Hefe Saccharomyces boulardii im Magen-Darm-Trakt ist für den Menschen ungefährlich. Sie wird unter anderem gefriergetrocknet in Kapseln zur Behandlung von Durchfall und zur Restaurierung der Darmflora nach oraler Einnahme von Antibiotika eingesetzt, wobei sie vermutlich im Darm die Vermehrung anderer, pathogener Erreger, durch welche Durchfälle verursacht werden könnten, zumindest zeitweilig durch ihre eigene starke Vermehrung hemmen. Saccharomyces boulardii hat im Gegensatz zu typischen Weinhefen eine ungewöhnlich hohe optimale Wachstumstemperatur von 37°C (Mansour-Ghanaei et al., World J Gastroenterol 2003 Aug; 9(8):1832-1833; McFarland et al., Microbial Ecology in Health and Disease 1993 July 6(4):157-171).

Lactobacillen zählen zu den gram-positiven Bakterien. Sie können durch Fermentation Milchsäure erzeugen und sind in der Regel für den Menschen nicht pathogen. Einige Arten der Lactobacillen werden in der Lebensmittelherstellung, beispielsweise in der Herstellung von Milchprodukten, genutzt. Lactobacillen können in der Lebensmittelindustrie allerdings auch als Schädlinge auftreten und unerwünschte Umsetzungen verursachen, wie zum Beispiel in der Bierbrauerei. Einige Lactobacillen bilden einen Teil der natürlichen Bakterienflora des Menschen, insbesondere des Magen-Darm-Trakts. So kann beispielsweise auch Lactobacillus plantarum im Magen-Darm-Trakt des Menschen auftreten. Lactobacillen tolerieren im allgemeinen niedrige pH-Werte besser als verwandte Gattungen, wie zum Beispiel Pediokokken, und wachsen auch bei pH-Werten von beispielsweise 4-5.

Im Gegensatz zu herkömmlichen Hefefermentationen von Früchten mit Weinhefen zur Produktion von Alkohol, die - entsprechend dem Temperaturoptimum der Weinhefen zur Alkoholproduktion - bei moderaten Temperaturen von 10 - 25°C erfolgen, hat es sich bei der Herstellung des erfindungsgemäßen polyphenolhaltigen Fermentationsprodukts überraschenderweise als besonders vorteilhaft erwiesen, eine Kulturtemperatur von 30 - 38°C während der Fermentation einzustellen. Es hat sich herausgestellt, dass die erforderlichen enzymatischen Reaktionen z.B. für die Depolymerisation und Deglykosylierung bei diesen Temperaturen am effektivsten ablaufen. Die Verwendung von Saccharomyces boulardii hat sich daher überraschenderweise erfindungsgemäß als besonders vorteilhaft erwiesen, da diese Hefe besonders hohe Temperaturen von über 30°C für ein optimales Wachstum preferiert. Zudem erwies es sich überraschenderweise, dass das Bakterium Lactobacillus plantarum, sowie auch Lactobacillus paraplantarum, Lactobacillus pentosus und Lactobacillus acidophilus, zur Fermentation von Granatapfelsaft unter Erhalt niedermolekularer Polyphenole geeignet sind. Diese Lactobacillen verfügen offensichtlich über eine geeignete Enzymausstattung. Es muss allerdings zum Start der Fermentation eine ausreichende Keimkonzentration an Lactobacillen verwendet und für günstige Wachstumsbedingungen (Temperatur, pH-Wert, Zuckergehalt) der Lösung gesorgt werden, da Granatapfelsaft antibakterielle Polyphenole enthält, die das bakterielle Wachstum hemmen. Die Enzymausstattung dieser Lactobacillen ergänzt außerdem die Enzymausstattung der Hefe Saccharomyces boulardii in besonders geeigneter Weise für die Herstellung des erfindungsgemäßen Fermentationsprodukts im Rahmen einer Ko-Fermentation.

Durch die Fermentation mit Saccharomyces boulardii und Lactobacillus plantarum / paraplantarum / pentosus / acidophilus wird der intestinale Verdauungsprozeß außerhalb des Körpers in gewisser Weise nachvollzogen, so daß auch bei Personen mit ungeeigneter Darmflora eine bessere Bioverfügbarkeit der Granatapfelpolyphenole erreicht wird. Solche ganzheitlichen Granatapfelprodukte mit niedermolekularen Polyphenolmetaboliten, die in einem darmähnlichen Milieu fermentiert wurden, sind gegenwärtig nicht bekannt.

Ein solches fermentiertes Granatapfelprodukt eignet sich sehr gut zum allgemeinen Zellschutz und zur Erhaltung der Zellgesundheit, in pharmakologischer Dosierung auch als Arzneimittel, das zur adjuvanten Therapie und Prävention von Krebserkrankungen (insbesondere Prostata-, Mamma-, Colon- und/oder Lungenkarzinom; insbesondere auch unterstützend und synergistisch bei einer Chemotherapie und/oder Strahlentherapie), benignen Prostatahyperplasie, arteriosklerotischen Gefäßveränderungen, koronarer Herzkrankheit, Zerebralsklerose, Hypercholesterinämie, chronischen entzündlichen Erkrankungen wie z.B. Rheumatoider Arthritis, Morbus Alzheimer, Morbus Parkinson, Multipler Sklerose und/oder Mikro- und Makroangiopathien bei Diabetes mellitus, geeignet ist.

Die erfindungsgemäßen Fermentationsprodukte können darüber hinaus mit einem gegenüber dem natürlichen Zuckergehalt des Granatapfelsafts deutlich reduzierten Zuckergehalt erhalten werden. Dies ermöglicht es beispielsweise auch Diabetikern, von den fermentierten Granatapfelpolyphenolen zu profitieren. Für Diabetiker ist dies besonders interessant, da für sie ein im Vergleich zum Bevölkerungsdurchschnitt stark erhöhtes Risiko für Mikro- und Makroangiopathien besteht, welches auf ernährungsphysiologische Weise durch die Granatapfelpolyphenole, wie in Studien gezeigt, reduziert werden kann. Das hierfür offensichtlich erforderliche Spektrum an Inhaltsstoffen ist überwiegend im Granatapfelmark enthalten, welches allerdings sehr zuckerhaltig und damit ohne vorherige Reduktion des Zuckergehalts für Diabetiker eingeschränkt geeignet ist. Mit dem erfindungsgemäßen Fermentationsprodukt wird es ermöglicht, den natürlichen Zuckergehalt des Granatapfelsafts stark zu reduzieren, jedoch gleichzeitig eine große Bandbreite an natürlichen Inhaltsstoffen des Granatapfelmarks und deren Derivaten zu erhalten.

Die erfindungsgemäßen Fermentationsprodukte weisen bevorzugt einen Zuckergehalt, gemessen als Anteil an Glucose und Fructose, von insgesamt maximal 2 Gew.-%, bezogen auf das Gesamtgewicht des Fermentationsansatzes nach Ende des Fermentationsprozesses und vor einer eventuellen Weiterverarbeitung, auf. Besonders bevorzugt weisen die erfindungsgemäßen Fermentationsprodukte einen Zuckergehalt von insgesamt maximal 1 Gew.%, ganz besonders bevorzugt von maximal 0,25 Gew.-%, gemessen als Anteil an Glucose und Fructose und bezogen auf das Gesamtgewicht des Fermentationsansatzes nach Ende des Fermentationsprozesses und vor einer eventuellen Weiterverarbeitung, auf.

Die erfindungsgemäßen Fermentationsprodukte enthalten bevorzugt 0,25 bis 4 Gew.-%, bezogen auf das Gesamtgewicht des Fermentationsansatzes nach Ende des Fermentationsprozesses und vor einer eventuellen Weiterverarbeitung, eines oder mehrerer Polyphenole. Besonders bevorzugt enthalten die erfindungsgemäßen Fermentationsprodukte 0,5 bis 3 Gew.-%, ganz besonders bevorzugt 0,7 bis 2,5 Gew.-%, jeweils bezogen auf das Gesamtgewicht des Fermentationsansatzes nach Ende des Fermentationsprozesses und vor einer eventuellen Weiterverarbeitung, eines oder mehrerer Polyphenole. Ein sehr geringer Polyphenolgehalt kann die Wirksamkeit und ernährungsphysiologische Qualität des Fermentationsprodukts einschränken. Bei einem sehr hohen Polyphenolgehalt kann es zu einer unerwünscht starken antimikrobiellen Wirkung im Fermentationsansatz kommen, die eine Fermentation unmöglich macht. Der Gehalt an Polyphenolen wird nach dem in der Wein- und Saftindustrie gebräuchlichen Folin-Ciocalteu-Verfahren unter Verwendung von Gallussäure als Bezugssubstanz bestimmt.

Bevorzugt liegt in dem erfindungsgemäßen Fermentationsprodukt ein größerer Anteil an Polyphenolen mit geringerem Molekulargewicht vor als in dem Fermentationsansatz zu Beginn der Fermentation. Das Molekulargewicht der Polyphenole kann beispielsweise mittels Massenspektrometrie nach vorheriger HPLC-Trennung bestimmt werden.

Bevorzugt enthält das erfindungsgemäße Fermentationsprodukt Polyphenole ausgewählt aus der Gruppe bestehend aus Gallussäure und/oder Derivaten der Gallussäure (z.B. Gallotannine); Ellagsäure und/oder Derivaten und/oder Metaboliten der Ellagsäure und von Ellagitanninen; Flavonoiden wie beispielsweise Quercetin und/oder Metaboliten und/oder Derivaten der Flavonoide, Anthocyanen und/oder Anthocyanidinen. Insbesondere bevorzugt enthält das erfindungsgemäße Fermentationsprodukt Ellagsäure, Derivate der Ellagsäure, Ellagsäuremetaboliten und/oder Ellagitannine.

Bevorzugt weisen die in dem erfindungsgemäßen Fermentationsprodukt enthaltenen Polyphenole einen deutlich reduzierten Anteil an glykosylierten Verbindungen auf. Die verschiedenen Polyphenole können beispielsweise durch chromatographische Verfahren, wie z.B. HPLC oder auch eine Kombination von HPLC und Massenspektrometrie, voneinander getrennt und identifiziert werden.

Ein weiterer Gegenstand der Erfindung ist ein Verfahren zur Herstellung eines Fermentationsproduktes, umfassend die Fermentation eines Fermentationsansatzes enthaltend Granatapfelsaft, wobei zur Fermentation die Hefe Saccharomyces boulardii und mindestens eine Art von Lactobacillen, ausgewählt aus der Gruppe bestehend aus den Arten Lactobacillus plantarum, Lactobacillus paraplantarum, Lactobacillus pentosus und Lactobacillus acidophilus, eingesetzt wird.

Zur Fermentation wird die Hefe Saccharomyces boulardii und mindestens eine Art von Lactobacillen, ausgewählt aus der Gruppe bestehend aus den Arten Lactobacillus plantarum, Lactobacillus paraplantarum, Lactobacillus pentosus und Lactobacillus acidophilus, eingesetzt. Insbesondere ist eine Ko-Fermentation unter Einsatz der Hefe Saccharomyces boulardii und mindestens eine Art von Lactobacillen, ausgewählt aus der Gruppe bestehend aus den Arten Lactobacillus plantarum, Lactobacillus paraplantarum, Lactobacillus pentosus und Lactobacillus acidophilus, ein Verfahren der Erfindung.

Die Fermentation kann im allgemeinen entweder zunächst in Gegenwart der Hefe Saccharomyces boulardii und daran anschließend in Gegenwart mindestens einer Art von Lactobacillen, ausgewählt aus der Gruppe bestehend aus den Arten Lactobacillus plantarum, Lactobacillus paraplantarum, Lactobacillus pentosus und Lactobacillus acidophilus; oder zunächst in Gegenwart mindestens einer Art von Lactobacillen, ausgewählt aus der Gruppe bestehend aus den Arten Lactobacillus plantarum, Lactobacillus paraplantarum, Lactobacillus pentosus und Lactobacillus acidophilus, und daran anschließend in Gegenwart der Hefe Saccharomyces boulardii; oder als Ko-Fermentation in gleichzeitiger Gegenwart der Hefe Saccharomyces boulardii und mindestens einer Art von Lactobacillen, ausgewählt aus der Gruppe bestehend aus den Arten Lactobacillus plantarum, Lactobacillus paraplantarum, Lactobacillus pentosus und Lactobacillus acidophilus, erfolgen. Bevorzugt erfolgt die Fermentation als Ko-Fermentation in gleichzeitiger Gegenwart der Hefe Saccharomyces boulardii und mindestens einer Art von Lactobacillen, ausgewählt aus der Gruppe bestehend aus den Arten Lactobacillus plantarum, Lactobacillus paraplantarum, Lactobacillus pentosus und Lactobacillus acidophilus.

In einer weiteren bevorzugten Ausführungsform wird nach einer etwa 1-3-tägigen Anfangsfermentation mit Saccharomyces boulardii oder einem anderen geeigneten Mikroorganismus anschließend mindestens eine Art von Lactobacillen, ausgewählt aus der Gruppe bestehend aus den Arten Lactobacillus plantarum, Lactobacillus paraplantarum, Lactobacillus pentosus und Lactobacillus acidophilus, zur weiteren Fermentation zugegeben.

Bevorzugt wird unter den Lactobacillen die Art Lactobacillus paraplantarum ausgewählt.

Ebenfalls bevorzugt wird unter den Lactobacillen die Art Lactobacillus plantarum ausgewählt.

Bevorzugt erfolgt die Fermentation als Ko-Fermentation in gleichzeitiger Gegenwart der Hefe Saccharomyces boulardii und Lactobacillus plantarum. Ganz besonders bevorzugt erfolgt die Fermentation als Ko-Fermentation in gleichzeitiger Gegenwart der Hefe Saccharomyces boulardii und Lactobacillus paraplantarum.

Zur Fermentation können beispielsweise Keimkonzentrationen von 2 x 10⁹ - 2 x 10¹¹ Keimen Saccharomyces boulardii pro 10 I Fermentationsansatz und/oder 2 x 10¹⁰ - 2 x 10¹³ Keimen der Lactobacillen pro 10 I Fermentationsansatz eingesetzt werden. Gegebenenfalls wird zunächst ein Inokulum mit einer erhöhten Keimkonzentration hergestellt, welches dann dem restlichen Fermentationsansatz zugegeben wird.

Zur Fermentation können daneben auch eine oder mehrere weitere, von den genannten Arten (Saccharomyces boulardii, Lactobacillus plantarum, Lactobacillus paraplantarum, Lactobacillus pentosus und Lactobacillus acidophilus) verschiedene Arten von Mikroorganismen zusätzlich eingesetzt werden. Beispielsweise können dem Fermentationsansatz zusätzlich zu Saccharomyces boulardii und Lactobacillus plantarum, Lactobacillus paraplantarum, Lactobacillus pentosus bzw. Lactobacillus acidophilus auch noch eine oder mehrere Arten von Weinhefen (z.B. Saccharomyces cerevisiae, Saccharomyces ellipsoides, Saccharomyces uvarum, Saccharomyces bayanus, Zygosaccharomyces) wie Champagnerhefe, Portweinhefe, Bordeauxhefe, Burgunderhefe oder Sherryhefe, und/oder ernährungsphysiologisch unbedenkliche Pilze wie z.B. Aspergillus oryzae zugefügt werden.

Der Granatapfelsaft kann durch Auspressen der zerkleinerten Früchte gewonnen werden. Beispielsweise können die geschälten Früchte mit einem Entsafter der Marke Champion der Firma Plastaket Manufacturing Co. (Lodi 95240, Kalifornien, USA; deutscher Importeur: Keimling Naturkost GmbH, 21614 Buxtehude) entsaftet werden. Der Granatapfelsaft kann aus den Früchten auch auf sonstige geeignete Arten gewonnen werden. Bevorzugt wird der Granatapfelsaft aus geschälten Granatäpfeln gewonnen.

Der Fermentationsansatz kann auch mindestens einen weiteren Bestandteil des Granatapfels, beispielsweise Blätter, Saftkonzentrat, Püree, Schalen, weiße Trennmembranen, Blüten und/oder Extrakte daraus, enthalten. Unter Blättern werden die grünen Blätter des Granatapfelbaums verstanden. Unter weißen Trennmembranen werden die weißen Trennwände verstanden, welche die von rotem Fruchtfleisch umhüllten Samen umgeben. Unter Blüten werden die roten Blüten des Granatapfelbaums verstanden.

Der Fermentationsansatz kann neben Granatapfelsaft auch Fruchtsaft und/oder Fruchtbestandteile einer oder mehrerer Pflanzen, vorzugsweise ausgewählt aus der Gruppe bestehend aus roten Trauben, Acai-Beeren, Amlabeeren, Aroniabeeren, Himbeeren, Erdbeeren, Holunderbeeren, Schlehen, schwarzen Johannisbeeren und schwarzen Kirschen, enthalten.

Der Fermentationsansatz weist zu Beginn der Fermentation vorzugsweise einen Zuckergehalt, gemessen als Anteil an Glucose und Fructose, von insgesamt 8 - 17 Gew.-%, besonders bevorzugt von 13 - 15 Gew.-%, jeweils bezogen auf das Gesamtgewicht des Fermentationsansatzes zu Beginn der Fermentation, auf. Bei einem zu niedrigen Zuckergehalt liegen im Fermentationsansatz nicht genügend Nährstoffe für die eingesetzten Mikroorganismen vor. Bei einem zu hohen Zuckergehalt kann es zu einer erheblichen Produktion von Alkohol kommen, welcher sich ebenfalls wachstumshemmend auf Hefen und Lactobacillen und gegebenenfalls weitere eingesetzte Mikroorganismen auswirken kann.

Der Fermentationsansatz weist vorzugsweise einen pH-Wert von 3 bis 6, besonders bevorzugt von 4 bis 5, auf. Der pH-Wert des Fermentationsansatzes kann beispielsweise durch Zugabe von Natriumhydrogencarbonat, Calciumcarbonat und/oder Kaliumcarbonat eingestellt und gegebenenfalls während des Fermentationsprozesses korrigiert werden. Ein Ansäuern des Fermentationsansatzes ist in der Regel nicht erforderlich, kann aber bei Bedarf beispielsweise durch Zugabe von Natriumcitrat erfolgen. Bei einem zu hohen oder zu niedrigen pH-Wert des Fermentationsansatzes sind Wachstum und Stoffwechsel der eingesetzten Mikroorganismen nicht optimal zur Herstellung des erfindungsgemäßen Fermentationsprodukts.

Als Fermentationsansatz wird das wäßrige Gemisch bezeichnet, welches entweder zu Beginn der Fermentation vorliegt und Granatapfelsaft, die Hefe Saccharomyces boulardii und mindestens eine Art von Lactobacillen, ausgewählt aus der Gruppe bestehend aus den Arten Lactobacillus plantarum, Lactobacillus paraplantarum, Lactobacillus pentosus und Lactobacillus acidophilus, sowie gegebenenfalls weitere Bestandteile enthält, sowie auch das wäßrige Gemisch, das hieraus während und nach der Fermentation durch den Fermentationsprozess entsteht.

Die Fermentation erfolgt vorzugsweise bei einer Temperatur von 30 - 38°C, besonders bevorzugt 35 - 37°C, des Fermentationsansatzes. Bei einer zu hohen oder zu niedrigen Temperatur des Fermentationsansatzes sind Wachstum und Stoffwechsel der eingesetzten Mikroorganismen nicht optimal für die Herstellung des erfindungsgemäßen Fermentationsprodukts.

Die Fermentation erfolgt vorzugsweise über einen Zeitraum von 3 bis 8 Tagen, besonders bevorzugt von 4 bis 6 Tagen. Der Endpunkt der Fermentation ist üblicherweise erreicht, wenn, infolge des durch die Stoffwechselaktivität der Mikroorganismen reduzierten Zuckergehalts des Fermentationsansatzes, die Kohlendioxidproduktion durch die verwendeten Mikroorganismen stark abgenommen hat und kaum noch oder gar nicht mehr beobachtet wird.

An die Fermentation können sich ein oder mehrere weitere Verfahrensschritte, bevorzugt eine Pasteurisierung, eine Lyophilisierung und/oder eine Micellierung in Liposomen, anschließen.

Soll das Fermentationsprodukt abgetötete Mikroorganismen enthalten, so schließt sich vorzugsweise eine Pasteurisierung und gegebenenfalls weitere Verfahrensschritte an. Soll das Fermentationsprodukt noch lebende Mikroorganismen enthalten, so entfällt vorzugsweise eine Pasteurisierung. Eine Pasteurisierung kann nach den dem Fachmann bekannten, geeigneten Methoden durchgeführt werden. Beispielsweise kann die Pasteurisierung durch kurzzeitiges Erwärmen des Fermentationsproduktes auf 60 - 90°C erfolgen. In einer möglichen Variante kann das Fermentationsprodukt zur Pasteurisierung für einige Sekunden auf etwa 85°C erwärmt werden. Vorzugsweise enthält das Fermentationsprodukt abgetötete Mikroorganismen.

In einer bevorzugten Ausführungsform schließt sich an die Fermentation nach einer Pasteurisierung eine Lyophilisierung des Fermentationsprodukts an. Nach der Lyophilisierung enthalten die erfindungsgemäßen Fermentationsprodukte bevorzugt 10 bis 30 Gew.-%, noch bevorzugter 15 bis 25 Gew-%, bezogen auf das Lyophilisat, eines oder mehrerer Polyphenole. Der Gehalt an Polyphenolen wird nach dem in der Wein- und Saftindustrie gebräuchlichen Folin-Ciocalteu-Verfahren unter Verwendung von Gallussäure als Bezugssubstanz und nach Rekonstituierung des Lyophilisats in destillliertem Wasser bestimmt. Die Lyophilisierung kann nach den dem Fachmann bekannten, geeigneten Methoden durchgeführt werden. Es können handelsübliche industrielle Gefriertrockner zur Lyophilisierung eingesetzt werden. Vorzugsweise werden zur Lyophilisierung keine kryoprotektiven Hilfsstoffe oder Trägersubstanzen zugesetzt.

Eine Micellierung in Liposomen erhöht die Lipophilie des Endprodukts. Bevorzugt erfolgt vor einer Micellierung in Liposomen eine Lyophilisierung des Fermentationsprodukts. Die Micellierung in Liposomen kann nach den dem Fachmann bekannten, geeigneten Methoden erfolgen. Als liposomenbildende, amphiphile Verbindungen können beispielsweise geeignete Phospholipide eingesetzt werden.

Dem Fermentationsansatz oder dem Fermentationsprodukt können vor oder nach einer eventuellen Pasteurisierung, Lyophilisierung und/oder Micellierung in Liposomen weitere Substanzen zugesetzt werden. Beispielsweise können eine oder mehrere der Substanzen ausgewählt aus der Gruppe bestehend aus Tributyrin, Butyrat, Curcumin und Granatapfelsamenöl, zugesetzt werden. Diese genannten Substanzen können ebenso wie die Granatapfelpolyphenole die Redifferenzierung von Krebszellen induzieren und werden bevorzugt in einer solchen Menge zugesetzt, dass im Endprodukt eine für diese Wirkung ausreichende Substanzkonzentration vorliegt.

Die technische Durchführung der Fermentation weist keine sonstigen Besonderheiten auf, sondern kann nach den dem Fachmann bekannten und geeigneten Methoden erfolgen. Beispielsweise kann die Fermentation in einem geeigneten Behälter mit einem CO₂-Auslaßventil unter Sauerstoffausschluß durchgeführt werden. Tritt gegen Ende der Fermentation ein Bodensatz auf, kann ein gelegentliches Umrühren des Fermentationsansatzes durchgeführt werden.

Die durch eines oder mehrere der oben beschriebenen Verfahren erhaltenen Fermentationsprodukte sind ebenfalls Gegenstand der Erfindung.

Ein weiterer Gegenstand der Erfindung ist die Verwendung des erfindungsgemäßen Fermentationsprodukts zur Herstellung eines Lebensmittels, eines Nahrungsergänzungsmittels, eines diätetischen Lebensmittels oder eines Kosmetikums. Bevorzugt wird das erfindungsgemäße Fermentationsprodukt zur Herstellung eines Lebensmittels, eines Nahrungsergänzungsmittels oder eines diätetischen Lebensmittels verwendet. Ganz besonders bevorzugt wird das erfindungsgemäße Fermentationsprodukt zur Herstellung eines Nahrungsergänzungsmittels verwendet.

Als Arzneimittel werden Mittel verstanden, die die Bestimmung haben, Krankheiten, Leiden oder Körperschäden zu heilen, zu lindern, zu verhüten oder zu erkennen oder die Beschaffenheit, den Zustand oder die Funktionen des Körpers oder seelische Zustände zu beeinflussen, und eine überwiegend pharmakologische Wirkung aufweisen. Lebensmittel sind keine Arzneimittel.

Demgegenüber zählen Nahrungsergänzungsmittel und diätetische Lebensmittel zu den Lebensmitteln. Lebensmittel sind Stoffe, die dazu bestimmt sind, in unverändertem oder verarbeiteten Zustand von Menschen verzehrt zu werden, ausgenommen Stoffe, die überwiegend zu anderen Zwecken als zur Ernährung oder zum Genuß bestimmt sind. Lebensmittel weisen daher eine überwiegend ernährungsphysiologische Wirkung auf. Diätetische Lebensmittel sind für eine besondere Ernährung bestimmt und entsprechen besonderen Ernährungserfordernissen von Verbrauchern, deren Verdauungs- oder Resorptionsprozeß oder Stoffwechsel gestört ist oder die sich in besonderen physiologischen Umständen befinden oder die gesunde Säuglinge oder Kleinkinder sind. Von den diätetischen Lebensmitteln unterscheiden sich Nahrungsergänzungsmittel dadurch, dass Nahrungsergänzungsmittel nicht einem besonderen Ernährungszweck dienen, sondern die allgemeine Ernährung ergänzen, z.B. als ein Konzentrat von Nährstoffen oder sonstigen Stoffen mit ernährungsspezifischer oder physiologischer Wirkung.

Kosmetika sind Stoffe oder Zubereitungen aus Stoffen, die ausschließlich oder überwiegend dazu bestimmt sind, äußerlich am Körper des Menschen oder in seiner Mundhöhle zur Reinigung (z.B. Seifen, Haarwaschmittel, Zahnputzmittel), zum Schutz, zur Erhaltung eines guten Zustandes (z.B. Hautcremes, Lotionen), zur Parfümierung, zur Veränderung des Aussehens (z.B. Bräunungsmittel, Haarfärbemittel) und/oder dazu angewendet zu werden, den Körpergeruch zu beeinflussen (z.B. Deosticks). Es können somit Produkte zur äußerlichen Anwendung sein, wie beispielsweise Salben, Cremes oder Sprühlösungen.

Werden unter Verwendung des erfindungsgemäßen Fermentationsprodukts Arzneimittel hergestellt, so kann es sich um Arzneimittel zur innerlichen oder äußerlichen Anwendung handeln. Bevorzugt handelt es sich in diesem Fall um ein Mittel zur innerlichen Anwendung, besonders bevorzugt zur peroralen oder rektalen Anwendung.

Bei den unter Verwendung des erfindungsgemäßen Fermentationsprodukts hergestellten Lebensmitteln, Nahrungsergänzungsmitteln und diätetischen Lebensmitteln handelt es sich um Mittel zur innerlichen Anwendung. Besonders bevorzugt werden die erfindungsgemäßen Fermentationsprodukte zur Herstellung von Nahrungsergänzungsmitteln und diätetischen Lebensmitteln zur peroralen Anwendung verwendet.

Die unter Verwendung der erfindungsgemäßen Fermentationsprodukte hergestellten Lebensmittel, Nahrungsergänzungsmittel, diätetischen Lebensmittel, Arzneimittel oder Kosmetika können verschiedene Darreichungsformen aufweisen. Bevorzugt handelt es sich bei den unter Verwendung der erfindungsgemäßen Fermentationsprodukte hergestellten Nahrungsergänzungsmitteln, diätetischen Lebensmitteln, Arzneimitteln oder Kosmetika um Mittel in Form von Tropfen, eines Saftes, eines Flüssigkonzentrats, einer Lösung, eines Elixiers, von Kapseln, von Tabletten, von Dragees, von Pastillen, von Globuli, eines Granulates, von Ampullen, von Suppositorien, von Klistieren, eines Pulvers oder von Brausetabletten oder um ein Mittel zur äußeren Anwendung, beispielsweise als Salbe, Creme oder Sprühlösung. Besonders bevorzugt handelt es sich um Mittel in Form eines Pulvers oder von Kapseln.

Bevorzugt handelt es sich bei ernährungsphysiologischer Dosierung des Fermentationsprodukts um ein Nahrungsergänzungsmittel oder diätetisches Lebensmittel zur allgemeinen Gesunderhaltung, insbesondere von Herz, Blutgefäßen und Prostata; oder bei pharmakologischer Dosierung um ein Arzneimittel, das insbesondere zur adjuvanten Therapie und Prävention von Krebserkrankungen (insbesondere Prostata-, Mamma-, Colon- und/oder Lungenkarzinom; insbesondere auch unterstützend und synergistisch bei einer Chemotherapie und/oder Strahlentherapie), benignen Prostatahyperplasie, arteriosklerotischen Gefäßveränderungen, koronarer Herzkrankheit, Zerebralsklerose, Hypercholesterinämie, chronischen entzündlichen Erkrankungen wie z.B. Rheumatoider Arthritis, Morbus Alzheimer, Morbus Parkinson, Multipler Sklerose und/oder Mikro- und Makroangiopathien bei Diabetes mellitus, geeignet ist.

Ein weiterer Gegenstand der Erfindung sind Lebensmittel, Nahrungsergänzungsmittel, diätetisches Lebensmittel oder Kosmetika, die ein erfindungsgemäßes Fermentationsprodukt und/oder ein nach dem erfindungsgemäßen Verfahren erhaltenes Fermentationsprodukt enthalten.

Im folgenden wird die Erfindung durch Ausführungsbeispiele näher erläutert.

### Ausführungsbeispiel 1

Zunächst werden zwei Inocula hergestellt:
Inoculum 1: Saccharomyces boulardii
   1 g Saccharomyces boulardii (2 x 10¹⁰ lyophilisierte Keime; im Handel erhältlich, beispielsweise in Apotheken) wird in 1 Liter Granatapfelsaft bei 36°C für 24 h inoculiert.
Inoculum 2: Lactobacillus plantarum
   0,5 g Lactobacillus plantarum (2 x 10¹¹ lyophilisierte Keime; im Handel erhältlich, beispielsweise von Danisco) wird in 1 Liter eines Gemisches aus 300 ml Wasser und 700 ml Granatapfelsaft bei einem pH-Wert von etwa 5 für 24 h bei 36°C inoculiert.

Dann wird der Fermentationsansatz hergestellt und die Fermentation durchgeführt. Hierzu werden die beiden Inocula in einem geeigneten Fermentationsgefäß mit CO₂ Auslaßventil mit 10 I Granatapfelsaft, der z.B. durch die Versaftung der geschälten Früchte mittels eines Entsafters der Marke Champion der Firma Plastaket Manufacturing Co. (Lodi 95240, Kalifornien, USA; deutscher Importeur: Keimling Naturkost GmbH, 21614 Buxtehude) gewonnen werden kann, gemischt. Der Granatapfelsaft sollte einen Zuckergehalt von ca. 14 g/l (Gesamtgehalt an Glucose und Fructose) aufweisen. Der pH-Wert des Fermentationsansatzes wird mit Natriumhydrogencarbonat oder Kaliumcarbonat auf pH 4,5 eingestellt. Der Fermentationsansatz wird über etwa 4-5 Tage bei einer Temperatur von 36°C unter Sauerstoffausschluß fermentiert, bis der Zuckergehalt auf unter 0,5% (5 g/l) gesunken ist und die CO₂-Produktion zum Stillstand gekommen ist.

Das erhaltene Fermentationsprodukt wird bevorzugt pasteurisiert und gefriergetrocknet oder auch weniger bevorzugt ohne Pasteurisierung mit den lebenden Keimen gefriergetrocknet.

### Ausführungsbeispiel 2

Das Inoculum 2 (Lactobacillus plantarum) aus Ausführungsbeispiel 1 wird in einem geeigneten Fermentationsgefäß mit CO₂-Auslaßventil mit 10 I Granatapfelsaft, der z.B. durch die Versaftung der geschälten Früchte mittels eines Entsafters der Marke Champion der Firma Plastaket Manufacturing Co. (Lodi 95240, Kalifornien, USA; deutscher Importeur: Keimling Naturkost GmbH, 21614 Buxtehude) gewonnen werden kann, gemischt. Der Granatapfelsaft sollte einen Zuckergehalt von ca. 14 g/l (Gesamtgehalt an Glucose und Fructose) aufweisen. Der pH-Wert des Fermentationsansatzes wird mit Natriumhydrogencarbonat oder Kaliumcarbonat auf pH 4,5 eingestellt. Der Fermentationsansatz wird über etwa 24 h bei einer Temperatur von 36°C unter Sauerstoffausschluß fermentiert. Danach wird das Inoculum 1 (Saccharomyces boutardii) aus Ausführungsbeispiel 1 hinzugegeben. Die Fermentation wird etwa 4-5 Tage bei einer Temperatur von 36°C unter Sauerstoffausschluß fortgesetzt, bis der Zuckergehalt auf unter 0,5% (5 g/l) gesunken ist und die CO₂-Produktion zum Stillstand gekommen ist.

Das erhaltene Fermentationsprodukt wird bevorzugt pasteurisiert und gefriergetrocknet oder auch weniger bevorzugt ohne Pasteurisierung mit den lebenden Keimen gefriergetrocknet.

## Patentansprüche

1. Fermentationsprodukt, erhältlich durch ein Verfahren umfassend die Fermentation eines Fermentationsansatzes enthaltend Granatapfelsaft, **dadurch gekennzeichnet, dass** zur Fermentation die Hefe Saccharomyces boulardii und mindestens eine Art von Lactobacillen, ausgewählt aus der Gruppe bestehend aus den Arten Lactobacillus plantarum, Lactobacillus paraplantarum, Lactobacillus pentosus und Lactobacillus acidophilus, eingesetzt wird.

2. Fermentationsprodukt nach Anspruch 1, **dadurch gekennzeichnet, dass** das Fermentationsprodukt 0,25 bis 4 Gew.-%, bezogen auf das Gesamtgewicht des Fermentationsansatzes nach Ende des Fermentationsprozesses und vor einer eventuellen Weiterverarbeitung, eines oder mehrerer Polyphenole enthält.

3. Fermentationsprodukt nach Anspruch 1 oder 2, **dadurch gekennzeichnet, dass** der Zuckergehalt des Fermentationsprodukts, gemessen als Anteil an Glucose und Fructose, insgesamt maximal 2 Gew.-%, bezogen auf das Gesamtgewicht des Fermentationsansatzes nach Ende des Fermentationsprozesses und vor einer eventuellen Weiterverarbeitung, beträgt.

4. Verfahren zur Herstellung eines Fermentationsproduktes nach Anspruch 1, umfassend die Fermentation eines Fermentationsansatzes enthaltend Granatapfelsaft, **dadurch gekennzeichnet, dass** zur Fermentation die Hefe Saccharomyces boulardii und mindestens eine Art von Lactobacillen, ausgewählt aus der Gruppe bestehend aus den Arten Lactobacillus plantarum, Lactobacillus paraplantarum, Lactobacillus pentosus und Lactobacillus acidophilus, eingesetzt wird.

5. Verfahren nach Anspruch 4, **dadurch gekennzeichnet, dass** die Fermentation entweder zunächst in Gegenwart der Hefe Saccharomyces boulardii und daran anschließend in Gegenwart mindestens einer Art von Lactobacillen, ausgewählt aus der Gruppe bestehend aus den Arten Lactobacillus plantarum, Lactobacillus paraplantarum, Lactobacillus pentosus und Lactobacillus acidophilus, erfolgt; oder dass die Fermentation zunächst in Gegenwart mindestens einer Art von Lactobacillen, ausgewählt aus der Gruppe bestehend aus den Arten Lactobacillus plantarum, Lactobacillus paraplantarum, Lactobacillus pentosus und Lactobacillus acidophilus, und daran anschließend in Gegenwart der Hefe Saccharomyces boulardii erfolgt; oder dass die Fermentation als Ko-Fermentation in gleichzeitiger Gegenwart der Hefe Saccharomyces boulardii und mindestens einer Art von Lactobacillen, ausgewählt aus der Gruppe bestehend aus den Arten Lactobacillus plantarum, Lactobacillus paraplantarum, Lactobacillus pentosus und Lactobacillus acidophilus, erfolgt.

6. Verfahren nach einem oder mehreren der vorhergehenden Ansprüche 4 bis 5, **dadurch gekennzeichnet, dass** unter den Lactobacillen die Art Lactobacillus paraplantarum ausgewählt wird.

7. Verfahren nach einem oder mehreren der vorhergehenden Ansprüche 4 bis 6, **dadurch gekennzeichnet, dass** der Fermentationsansatz mindestens einen weiteren Bestandteil des Granatapfels, beispielsweise Blätter, Saftkonzentrat, Püree, Schalen, weiße Trennmembranen, Blüten und/oder Extrakte daraus, enthält.

8. Verfahren nach einem oder mehreren der vorhergehenden Ansprüche 4 bis 7, **dadurch gekennzeichnet, dass** der Fermentationsansatz Fruchtsaft und/oder Fruchtbestandteile einer oder mehrerer Pflanzen, vorzugsweise ausgewählt aus der Gruppe bestehend aus roten Trauben, Acai-Beeren, Amlabeeren, Aroniabeeren, Himbeeren, Erdbeeren, Holunderbeeren, Schlehen, schwarzen Johannisbeeren und schwarzen Kirschen, enthält.

9. Verfahren nach einem oder mehreren der vorhergehenden Ansprüche 4 bis 8, **dadurch gekennzeichnet, dass** der Fermentationsansatz einen pH-Wert von 3 bis 6, vorzugsweise von 4 bis 5 aufweist.

10. Verfahren nach einem oder mehreren der vorhergehenden Ansprüche 4 bis 9, **dadurch gekennzeichnet, dass** die Fermentation bei einer Temperatur von 30 - 38°C, bevorzugt 35 - 37°C, des Fermentationsansatzes erfolgt.

11. Verfahren nach einem oder mehreren der vorhergehenden Ansprüche 4 bis 10, **dadurch gekennzeichnet, dass** der Fermentationsansatz zu Beginn der Fermentation einen Zuckergehalt, gemessen als Anteil an Glucose und Fructose, von insgesamt 8 - 17 Gew.-%, bevorzugt von 13 - 15 Gew.-%, jeweils bezogen auf das Gesamtgewicht des Fermentationsansatzes zu Beginn der Fermentation, aufweist.

12. Verfahren nach einem oder mehreren der vorhergehenden Ansprüche 4 bis 11, **dadurch gekennzeichnet, dass** sich an die Fermentation ein oder mehrere weitere Verfahrensschritte, bevorzugt eine Pasteurisierung, eine Lyophilisierung und/oder eine Micellierung in Liposomen, anschließen.

13. Verfahren nach einem oder mehreren der vorhergehenden Ansprüche 4 bis 12, **dadurch gekennzeichnet, dass** zur Fermentation eine oder mehrere weitere, von den genannten Arten Saccharomyces boulardii, Lactobacillus plantarum, Lactobacillus paraplantarum, Lactobacillus pentosus und Lactobacillus acidophilus verschiedene Arten von Mikroorganismen zusätzlich eingesetzt werden.

14. Verwendung eines Fermentationsprodukts nach einem oder mehreren der Ansprüche 1 bis 3 und/oder erhältlich nach einem oder mehreren der in den Ansprüchen 4 bis 13 beschriebenen Verfahren zur Herstellung eines Lebensmittels, eines Nahrungsergänzungsmittels, eines diätetischen Lebensmittels oder eines Kosmetikums.

15. Verwendung nach Anspruch 14, **dadurch gekennzeichnet, dass** es sich um ein Lebensmittel, um ein Nahrungsergänzungsmittel oder um ein diätetisches Lebensmittel handelt.

16. Lebensmittel, Nahrungsergänzungsmittel, diätetisches Lebensmittel oder Kosmetikum, enthaltend ein Fermentationsprodukt nach einem oder mehreren der Ansprüche 1 - 3 und/oder ein nach einem Verfahren nach einem oder mehreren der Ansprüche 4 - 13 erhaltenes Fermentationsprodukt.

## Claims

1. A fermentation product which can be obtained by means of a method comprising the fermentation of a ferment containing pomegranate juice, wherein the yeast *Saccharomyces boulardii* and at least one species of lactic acid bacilli, chosen from the group constituted of the species *Lactobacillus plantarum, Lactobacillus paraplantarum, Lactobacillus pentosus* and *Lactobacillus acidophilus,* are used for the fermentation.

2. The fermentation product as claimed in claim 1, which contains 0.25% to 4% of one or more polyphenols, relative to the total weight of the ferment at the end of the fermentation process and before any optional other transformation.

3. The fermentation product as claimed in claim 1 or 2, wherein the sugar content of the fermentation product, measured as percentage of glucose and of fructose, amounts to a maximum of 2% relative to the total weight of the ferment after the end of the fermentation process and before any optional other transformation.

4. A method for the production of a fermentation product as claimed in claim 1, comprising the fermentation of a ferment containing pomegranate juice, wherein the yeast *Saccharomyces boulardii* and at last one species of lactic acid bacilli, chosen from the group constituted of the species *Lactobacillus plantarum, Lactobacillus paraplantarum, Lactobacillus pentosus* and *Lactobacillus acidophilus,* are used for the fermentation.

5. The method as claimed in claim 4, wherein the fermentation is first carried out in the presence of the yeast *Saccharomyces boulardii* and immediately afterward in the presence of at least one species of lactic acid bacilli, chosen from the group constituted of the species *Lactobacillus plantarum, Lactobacillus paraplantarum, Lactobacillus pentosus* and *Lactobacillus acidophilus;* or wherein the fermentation is first carried out in the presence of at least one species of lactobacilli, chosen from the group of the species *Lactobacillus plantarum, Lactobacillus paraplantarum, Lactobacillus pentosus* and *Lactobacillus acidolphus,* and immediately afterward in the presence of the yeast *Saccharomyces boulardii;* or wherein the fermentation takes place as a cofermentation in the simultaneous presence of the yeast *Saccharomyces boulardii* and of at least one species of lactobacilli, chosen from the group constituted of the species *Lactobacillus plantarum, Lactobacillus paraplantarum, Lactobacillus pentosus* and *Lactobacillus acidophilus.*

6. The method as claimed in one or both of claims 4 and 5 above, wherein, among the lactobacilli, the species *Lactobacillus paraplantarum* is chosen.

7. The method as claimed in one or more of claims 4 to 6 above, wherein the ferment contains at least one other pomegranate component, for example leaves, juice concentrate, purée, peel, white membranes, flowers and/or extracts thereof.

8. The method as claimed in one or more of claims 4 to 7 above, wherein the ferment contains fruit juice and/or fruit components of one or more plants, preferably chosen from the group constituted of black grapes, acai berries, Indian gooseberries, aronia berries, raspberries, strawberries, elderberries, sloes, blackcurrant and black cherries.

9. The method as claimed in one or more of claims 4 to 8 above, wherein the ferment has a pH value of 3 to 6, preferably of 4 to 5.

10. The method as claimed in one or more of claims 4 to 9 above, wherein the fermentation takes place at a temperature of from 30 to 38°, or even better still from 35 to 37°.

11. The method as claimed in one or more of claims 4 to 10 above, wherein, at the beginning of the fermentation, the ferment has a sugar content, calculated as percentage of glucose and of fructose, of from 8% to 17%, or even better still from 13% to 15%, relative to the total weight of the ferment at the beginning of the fermentation.

12. The method as claimed in one or more of claims 4 to 11 above, wherein the fermentation follows one or more other steps, preferably a pasteurization, a lyophilization and/or a micellar transformation to liposomes.

13. The method as claimed in one or more of claims 4 to 12 above, wherein one or more species different than microorganisms of the species named *Saccharomyces boulardii, Lactobacillus plantarum, Lactobacillus paraplantarum, Lactobacillus pentosus* and *Lactobacillus acidophilus* is (are) also used for the fermentation.

14. The use of a fermentation product as claimed in one or more of claims 1 to 3 and/or obtained after one or more of the methods described in claims 4 to 13, for the production of a food product, of a food supplement, of a dietetic food product or of a cosmetic product.

15. The use as claimed in claim 14, wherein a food product, a food supplement or a dietetic food product is involved.

16. A food product, a food supplement, a dietetic food product or a cosmetic product, containing a fermentation product as claimed in one or more of claims 1 to 3 and/or a fermentation product obtained through the use of a method described in one or more of claims 4 to 13.

## Revendications

1. Produit de fermentation qui peut être obtenu par un procédé comprenant la fermentation d'un ferment contenant du jus de grenade, **caractérisé par le fait que** l'on utilise pour la fermentation la levure saccharomyces boulardii et au moins une espèce de bacilles lactiques choisie dans le groupe constitué des espèces lactobacillus plantarum, lactobacillus paraplantarum, lactobacillus pentosus et lactobacillus acidophilus

2. Produit de fermentation selon la revendication 1, **caractérisé par le fait que** ce produit contient 0,25 à 4 % d'un ou plusieurs polyphénols, par rapport au poids total du ferment à la fin du processus de fermentation et avant une éventuelle autre transformation.

3. Produit de fermentation selon les revendications 1 ou 2, **caractérisé par le fait que** la teneur en sucre du produit de fermentation, mesuré en pourcentage de glucose et de fructose s'élève à maximum 2 % par rapport au poids total du ferment après la fin du processus de fermentation et avant une éventuelle autre transformation.

4. Procédé de fabrication d'un produit de fermentation selon la revendication 1, comprenant la fermentation d'un ferment contenant du jus de grenade, **caractérisé par le fait qu'**on utilise pour la fermentation la levure saccharomyces boulardii et au moins une espèce de bacilles lactiques choisie dans le groupe constitué par les espèces lactobacillus plantarum, lactobacillus paraplantarum, lactobacillus pentosus et lactobacillus acidophilus.

5. Procédé selon la revendication 4, **caractérisé par le fait que** la fermentation se fait d'abord en présence de la levure saccharomyces boulardii et immédiatement après en présence d'au moins une espèce de bacilles lactiques choisie dans le groupe constitué par les espèces lactobacillus plantarum, lactobacillus paraplantarum, lactobacillus pentosus et lactobacillus acidophilus ; ou que la fermentation se fait d'abord en présence d'au moins une espèce de lactobacilles choisie dans le groupe des espèces lactobacillus plantarum, lactobacillus paraplantarum, lactobacillus pentosus et lactobacillus acidolphus et immédiatement après en présence de la levure saccharomyces boulardii ; ou que la fermentation a lieu comme co-fermentation en présence simultanée de la levure saccharomyces boulardii et d'au moins d'une espèce de lactobacilles choisie dans le groupe constitué par les espèces lactobacillus plantarum, lactobacillus paraplantarum, lactobacillus pentosus et lactobacillus acidophilus.

6. Procédé selon une ou plusieurs des revendications 4 à 5 ci-dessus, **caractérisé par le fait que** l'on choisit parmi les lactobacilles l'espèce lactobacillus paraplantarum

7. Procédé selon une ou plusieurs des revendications 4 à 6 ci-dessus, **caractérisé par le fait que** le ferment contient au moins un autre composant de la grenade, par exemple des feuilles, du concentré de jus, de la purée, de la peau, des membranes blanches, des fleurs et/ou des extraits de ceux-ci.

8. Procédé selon une ou plusieurs des revendications 4 à 7 ci-dessus, **caractérisé par le fait que** le ferment contient du jus de fruit et/ou des composants de fruit d'une ou de plusieurs plantes, de préférence choisies dans le groupe constitué par les raisins noirs, les baies d'acai, les baies d'amla, les baies d'aronia, les framboises, les fraises, les baies de sureau, les prunelles, le cassis et les cerises noires.

9. Procédé selon une ou plusieurs des revendications 4 à 8 ci-dessus, **caractérisé par le fait que** le ferment a une valeur pH de 3 à 6, de préférence de 4 à 5.

10. Procédé selon une ou plusieurs des revendications 4 à 9 ci-dessus, **caractérisé par le fait que** la fermentation a lieu par une température de 30 à 38°, ou mieux encore de 35 à 37°.

11. Procédé selon une ou plusieurs des revendications 4 à 10 ci-dessus, **caractérisé par le fait qu'**au début de la fermentation, le ferment a une teneur en sucre, calculée en pourcentage de glucose et de fructose de 8 à 17 % ou mieux encore de 13 à 15% par rapport au poids total du ferment au début de la fermentation.

12. Procédé selon une ou plusieurs des revendications 4 à 11 ci-dessus, **caractérisé par le fait qu'**à la fermentation succèdent une ou plusieurs autres étapes, de préférence une pasteurisation, une lyophilisation et/ou une transformation micellaire en liposomes.

13. Procédé selon une ou plusieurs des revendications 4 à 12 ci-dessus, **caractérisé par le fait que** l'on utilise en plus pour la fermentation une ou plusieurs espèces différentes de microorganismes des espèces appelées saccharomyces boulardii, lactobacillus plantarum, lactobacillus paraplantarum, lactobacillus pentosus et lactobacillus acidophilus.

14. Utilisation d'un produit de fermentation selon une ou plusieurs des revendications 1 à 3 et/ou obtenu après un ou plusieurs des procédés décrits dans les revendications 4 à 13 pour la fabrication d'un aliment, d'un complément alimentaire, d'un d'aliment diététique ou d'un produit cosmétique

15. Utilisation selon la revendication 14, **caractérisée par le fait qu'**il s'agit d'un aliment, d'un complément alimentaire ou d'un aliment diététique.

16. Aliment, complément alimentaire, aliment diététique ou produit cosmétique, contenant un produit de fermentation selon une ou plusieurs des revendications 1 à 3 et/ou un produit de fermentation obtenu par l'emploi d'un procédé décrit dans une ou plusieurs des revendications 4 à 13
